# EUROPEAN PATENT SPECIFICATION

(11) **EP 0 333 801 B1**
(45) Date of publication and mention of the grant of the patent: **18.10.1995**
(21) Application number: 88907901.8
(22) Date of filing: 05.08.1988
(51) Int. Cl.: G01N 33/543, G01N 33/545, G01N 33/552, G01N 33/53, C12Q 1/68

(54) **TEST STRIP FOR DIAGNOSIS BY MULTI-IMMUNOASSAY SYSTEM**
TESTSTREIFEN FÜR DIAGNOSE DURCH MULTI-IMMUNOASSAY SYSTEM
LANGUETTE DE DIAGNOSTIC PAR SYSTEME MULTI-IMMUNODOSAGE

(30) Priority: 05.08.1987 US 81874
(43) Date of publication of application: 27.09.1989
(73) Proprietor: Calypte, Inc., Berkeley, CA 94710 (US)
(72) Inventor: URNOVITZ, Howard, B., San Francisco, CA 94121 (US)
(74) Representative: Henkel, Feiler, Hänzel & Partner
(86) International application number: US8802684
(87) International publication number: WO8901158

(56) References cited:
- EP-A- 0 017 150
- EP-A- 0 093 613
- EP-A- 0 200 381
- EP-A- 0 201 716
- EP-A- 0 217 403
- EP-A- 0 253 464
- US-A- 4 299 916
- US-A- 4 540 659
- US-A- 4 558 013
- TRENDS IN BIOTECHNOLOGY, vol. 8, 1990, Elsevier Science Publishers Ltd., GB; T.D. GOTTFRIED et al., pp. 35-40#
- 5th INTERNATL. CONFERENCE ON AIDS, THE SCIENTIFIC AND SOCIAL CHALLENGE, Montreal, CD, 04-09 June 1989; H.B. UMOVITZ et al., abstract#
- BIOLOGICAL ABSTRACTS, vol. 79, no. 11, 01 June 1985, Philadelphia, PA (US); W.C. GOH, p. 368, no. 94949#
- BIOLOGICAL ABSTRACTS, vol. 84, no. 10, 15 September 1987, Philadelphia, PA (US); A.G. FISHER, p. 1160, no. 104387#

## Description

### Field of the Invention

This invention is in the field of immunodiagnostics. More specifically, this invention provides test strips, as well as kits containing, and methods employing, these strips for use in the immunological detection of analytes in aqueous liquids, particularly biological samples such as blood, urine, and the like.

### Background of the Invention

In recent years, the detection, or quantitative determination, or both, of analytes based upon reactions with immunological reagents has gained considerable importance, especially in the field of medical testing. Commonly, these methods involve contacting a sample suspected of containing the analyte with a material which exhibits specific immunologic reactivity with the analyte, for example, an antibody directed to an epitope present on the analyte. If the analyte is present in the sample, it specifically conjugates with the antibody to form a complex. A wide range of "developer" or "reporter" mechanisms have been proposed and are in use to indicate whether the conjugation reaction occurs. (See, for example, United States Patent Nos. 4,366,242, issued December 28, 1982 to Neumann, et al; 4,278,653, issued July 14, 1981 to Harris, et al.; and 4,208,479, issued June 17, 1980 to Zuk, et al.)

Such methods have become increasingly popular since the introduction of monoclonal antibodies (MAbs), which may be produced using the technology developed by Kohler & Milstein, "Continuous Cultures of Fused Cells Secreting Antibody of Preferred Specificity," Nature, (1975) 256: 495-7, and which have unique specificity for the analytes with which they conjugate. (See, for example, United States Patent No. 4,376,110, issued March 8, 1983 to David, et al.).

As these methods have evolved, there has been a parallel search for better ways to apply them on a day-to-day basis. This has led to a range of test devices, test kits, and the like. (See, for example, United States Patent Nos. 4,623,461, issued November 18, 1986 to Hossom, et al.; 3,888,629, issued June 10, 1975 to Bagshawe: 4,458,020, issued July 3, 1984 to Bohn, et al.; 4,496,654, issued January 29, 1985 to Katz, et al.; and 4,305,924, issued December 15, 1981 to Piasik, et al.)

European patent publication No. 253 464 discloses a method for providing internal reference for use in a sequential analyte-receptor assay for the determination of a target analyte in a sample. The assay uses a signal producing system and an analyte receptor capable of binding the target analyte at a discrete test zone on a solid phase. The method comprises binding a reference receptor, selected to bind with an analyte receptor conjugate capable of complexation with the target analyte, at a discrete reference zone separate from the discrete test zone on the solid phase.

Desirable characteristics of a test kit or device include the following:
1. The test device or kit should be easy to store and to use.
2. It should give unambiguous results without false positives or false negatives.
3. It should allow a multiplicity of samples to be screened in a short period.
4. Ideally, it should provide the user with positive indication that it has been used properly as confirmation that a false reading has not been obtained.
5. Preferably, it should allow a plurality of tests to be run simultaneously.
6. Preferably, the test can be configured to use whole blood or its fractionated components.

It is a primary object of the present invention to provide a test strip and a test kit based thereon which provides these desirable features.

### Summary of the Invention

The present invention provides a test strip for detecting, in a sample from a human subject, the presence of an antibody which comprises a solid support, the antigenic substance which binds to the antibody bound to a first discrete area on the solid support, an anti-(human antibody) antibody bound to a second discrete area on the solid support as a positive control, and an antibody directed against an antigen which does not naturally occur in human subjects bound to a third discrete area on the solid support as a negative control.

The invention additionally provides a test strip for detecting, in a sample from a human subject, the presence of an antigenic substance which comprises a solid support, an antibody-based reagent directed against the antigenic substance which substance is in an immune complex with a native human antibody thereto bound to a first discrete area on the solid support, an anti-(human antibody) antibody bound to a second discrete area on the solid support as a positive control, and an antibody directed against an antigen which does not naturally occur in human subjects bound to a third discrete area on the solid support as a negative control.

The invention also provides a method for detecting in a sample from a human subject the presence of an antigenic substance using the aforementioned test strips.

Thus, the present invention provides a test strip for immunologically detecting the presence of one or more analytes in aqueous specimens. The strip device comprises a solid support which has a plurality of discrete areas on its surface. At least one of these areas is a test area which carries a monoclonal antibody (or a combination selected from polyclonal antibody, mono- or divalent antibody fragment, hybrid antibody, heterobifunctional antibody, or genetically manipulated or cloned antibody) to an analyte to be detected or quantitatively determined. This antibody or combination is preferably conjugated to, or immobilized on, the surface of the support. At least one of the discrete areas on the test strip carries a positive control, i.e., a control material which produces a positive signal only when the test area has been contacted with the specimen. The positive control is an immunological control, such that the control event includes an immunological reaction between a species always present in the specimen and its immunologically specific partner immobilized in the positive control zone.

Moreover, the test strip includes at least one area carrying a negative control, that is, a control material which does not give rise to a detectable event in the presence of a normal specimen but does give rise to a detectable event when the sample is abnormal.

In another embodiment, this test strip contains a plurality of separate test areas containing different antibodies or combinations of antibodies so as to detect more than one analyte in the specimen.

In another embodiment, this test strip contains a plurality of separate test areas containing different analytes or combinations of analytes to detect antibody or other analytes.

A test kit can also be provided. This test kit includes the test strip just described and a test plate comprising a plurality of liquid holders, each shaped and sized to receive the test strip and to permit the test areas and positive control zone and negative control zone, if any, to simultaneously contact each of a sequence of liquids which are predeposited in the holders and at least one of which includes the test specimen.

In a presently preferred embodiment of this kit, the test plate comprises a plurality of liquid holders so as to permit a plurality of specimens to be tested at the same time, using a plurality of test strips.

### Description of the Figures

Figure 1 is a perspective view of a test strip in accordance with the invention, showing its various zones or areas in semischematic form;
Figure 2 is a perspective and semischematic view of another embodiment of a test strip of this invention, together with a sample test plate;
Figure 3 is a perspective view of a test plate adapted to permit multiple specimens to be tested simultaneously; and
Figure 4 is a block diagram of a typical test protocol which may be employed according to the present invention.

### Detailed Description of the Invention

This invention provides a test strip for detecting, in a sample from a subject, particularly a human subject the presence of an antibody. This test strip comprises a solid support, the antigenic substance which binds to the antibody bound to a first discrete area on the solid support, an anti-(human antibody) antibody bound to a second discrete area on the solid support as a positive control, and an antibody directed against an antigen which does not naturally occur in human subjects bound to a third discrete area on the solid support as a negative control. Such a test strip may also be readily adapted so as to quantitatively determine the amount or concentration of the antigenic substance present in the sample.

This invention also provides a test strip which comprises all of the elements of the aforementioned test strip and additionally comprising an antibody-based reagent directed against an antigenic substance which substance is in an immune complex with a native human antibody thereto, bound to a fourth discrete area on the solid support.

In one embodiment of this invention, the antigenic substance is a virus or a viral protein. For example, the antigenic substance may be HIV-1, HIV-1 GAG protein, HIV-1 ENV glycoprotein, HIV-1 POL protein, HTLV-1 GAG protein, HTLV-1 ENV glycoprotein, HTLV-1 POL protein, or an epitope thereof.

In test strips according to this invention, the solid support may comprise glass fiber filter paper, nitrocellulose, scintered glass, plastic, synthetic polymer, cellulose, cellulose acetate, polytetrafluoroethylene, polyethylene, polypropylene, or polyvinylidine fluoride.

The antibody-based reagent comprises a monoclonal antibody, polyclonal antibody, mono- or divalent antibody fragment, hybrid antibody, heterofunctional antibody, or genetically manipulated or cloned antibody.

In a preferred embodiment of this invention the anti-human antibody on the test strip is a monoclonal antibody.

Similarly in a preferred embodiment of this invention, the antigen which does not naturally occur in human subjects and which is present on the test strip is a synthetic organic molecule, e.g., dinitrophenol.

In preferred embodiments of this invention one or more of the anti-(human antibody) antibody, the antibody directed against an antigen which does not naturally occur, or the antibody-based reagent is labeled, for example, labeled with a radioactive isotope, fluorophore, chromophore, or an enzyme which catalyzes a chemical reaction which produces a detectable product.

The invention also provides a test strip for detecting, in a sample from a human subject, the presence of an antigenic substance which comprises a solid support, an antibody-based reagent directed against the antigenic substance in an immune complex and native human antibody thereto bound to a first discrete area on the solid support, an anti-human antibody bound to a second discrete area on the solid support as a positive control, and an antibody directed against an antigen which does not naturally occur in human subjects bound to a third discrete area on the solid support as a negative control. As those skilled in the art will appreciate, such a test strip may be readily adapted so as to quantitatively determine the amount or concentration of the antigenic substance present in the sample.

The invention further provides a method for detecting in a sample from a human subject the presence of an antibody which comprises pretreating the sample to be tested so as to prevent non-specific and specific binding of substances present in the sample to proteins, including antibodies and recombinant antigens, present on the test strip, and thus prevent spurious results; contacting the resulting pretreated sample to be tested with the aforementioned test strip under conditions such that the antigenic substance bound to the test strip forms a complex with any antibody directed against it which is present in the sample and in the case of test strips onto which both the antigenic substance and the antibody-based reagent are bound, so that antigenic substance present in the sample binds to antibody thereto present on the test strip; thereafter treating the test strip to remove uncomplexed antibody directed against the antigenic substance; contacting the resulting, treated test strip with labeled antihuman antibody under conditions such that the antihuman antibody forms a complex with any human antibody bound to the test strip; detecting the presence of labeled antihuman antibody bound to the test strip and thereby the presence of, or antibody to, the antigenic substance and thus detecting the presence of the antigenic substance in the sample; and verifying the correctness of the detection so made by means of the positive and negative controls on the test strip.

The invention additionally provides a method of detecting in a sample from a human subject the presence of an antigenic substance which comprises pretreating the sample to be tested so as to prevent non-specific binding of substances present in the sample to proteins, including antibodies, present on the test strip and specific binding of human antibodies to the antigenic substance, and thus prevent spurious results; contacting the resulting pretreated sample with an aforementioned test strip under conditions such that the antibody-based reagent bound to the test strip forms a complex with any antigenic substance which is present in the sample; contacting the resulting, treated test strip with labeled antibody directed to the antigenic substance under conditions such that the labeled antibody forms a complex with any antigenic substance bound to the test strip; detecting the presence of labeled antibody directed to the antigenic substance bound to the test strip and thereby the presence of the antigenic substance in the sample; and verifying the correctness of the detection so made by means of the positive and negative controls on the test strip.

In preferred embodiments of the invention the hereinabove described method involves as the antigenic substance, a virus or viral protein, and as the sample, blood, serum, urine, or the like.

The invention also provides the aforementioned methods wherein the verification comprises contacting the test strip with labeled antihuman antibody under conditions such that the labeled antihuman antibody binds to human antibody bound to the test strip.

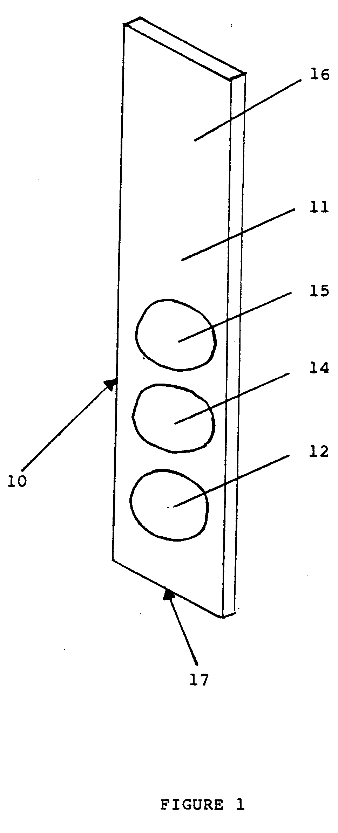

Thus, the present invention provides an improved test strip device. One embodiment of this device is shown as 10 in Figure 1. It includes a solid support 11 having a plurality of readable areas 12, 14 and 15. While support 11 is shown as a solid "dip stick" structure, it can be virtually any geometric shape, including cube, block, rod, cylinder, prism, polygon, spiral, sphere, or segmented combinations thereof. It can also be a carrier device holding materials such as plastic, metal, paper, or the like. It can be made of a wide range of materials including plastic, such as polystyrene or the like; metal; paper; glass fiber; filter paper; nitrocellulose; scintered glass; synthetic polymers; cellulose; cellulose acetate; polytetrafluoroethylene; polyethylene; polypropylene; polyvinylidine fluoride; or any other material including the materials which it comes in contact with during the test. Commonly, the materials deposited on its surface are adsorbed, chemically coupled, or otherwise bound to the surface of the test strip as described hereinafter.

Readable areas 12, 14 and 15 are arrayed separately on support 11 so as to give a plurality of potential signals when the strip is processed in the analysis method. At least one of the readable areas includes antibody which is immunoreactive with a test analyte in the test specimen. The readable areas may also include an analyte reactive with test antibody or test analyte. At least one of the readable areas includes a positive control which reacts with a test specimen to verify via a detectable signal when the test strip properly contacts a test specimen. The positive control is an immunologic positive control which undergoes immunologic reaction with a nonanalyte species in the specimen.

The readable areas can also include a negative control, namely, a discrete area containing one or more reagents that do not produce a reading with the specimen if the specimen is properly handled but which do give a reaction and produce a signal if the specimen is defective or had been mishandled.

The relative position of the positive control area and the antibody-containing areas may be important. Test strip 10 is used as a "dip stick." In use, the technician or automated test device grips or "holds" the strip by top end 16 and dips it into the liquid specimen and other reagents, lower end 17 first. This means that area 12 will contact these liquids before area 15 does. It is desirable, therefore, that the positive control, which gives a positive reading only when the specimen and reagents have properly contacted it be positioned as the last to be contacted or uppermost area, e.g., 15 in Figure 1. Conversely, the negative control, if present, should be positioned as the first to be contacted or bottommost, e.g., 12 in Figure 1.

The positive control and the negative controls are immunologic. That is, they preferably function by means of immunological reactions. Thus, for example, when the test specimens are human serum-based, a typical positive control could be a region designed to be treated with a common immunoglobulin always found in blood or its fractionated components.

The test reagent region (14 in Figure 1) is based on and includes at least one antibody specific for the analyte being tested for. A wide range of antibodies against a wide range of potentially important analytes have been described in the literature and may be used.

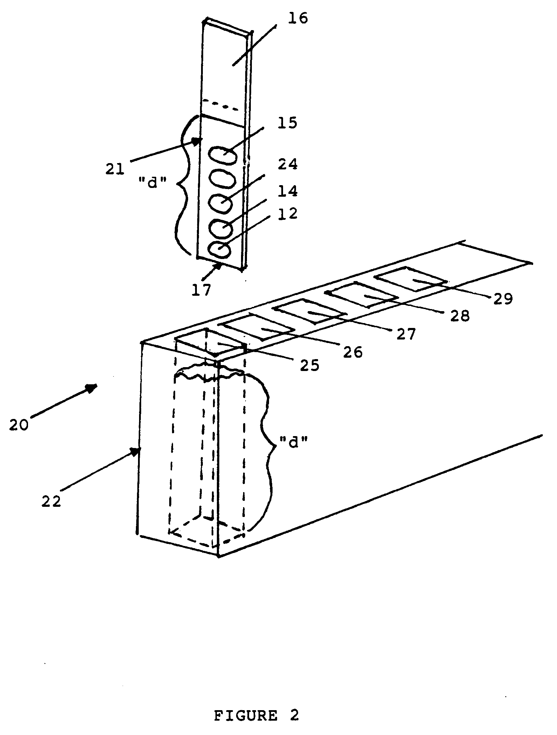

Turning to Figure 2, a variation on the test strip of Figure 1 is shown as 21. Strip 21 is shown as part of an overall analysis kit 20 which also includes a test plate 22. Strip 21 includes support 11 and readable areas 12, 14 and 15 as previously described and an additional readable area 24. Area 24 may be an additional control, an analyte, or an additional antibody-containing test region. Area 24 may contain a second antibody against the same antigen of area 14 so as to provide additional confirmation or sophistication to the test result or it may contain an antibody against a second antigen-analyte. These various reagents are generally immobilized in their various regions to prevent cross-contamination and the like, which will give ambiguous results. Antibody or most analytes are commonly immobilized on plastics, carbohydrate polymers, adsorbent fibers, polymer-coated metal beads, silica gel, paper, glass filters, or the like, by either direct adsorption (incubation for limited times or until the reagent solution is completely dry) or by chemical coupling to reactive groups on the solid phase. The optimal conditions are unique for each reagent.

In a typical application, the test strip 21 is employed in concert with a test plate such as 22. Test plate 22 has several characteristics. For one, it contains a plurality of liquid storage wells such as 25, 26, 27, 28 and 29. The exact number is selected to accommodate the various steps required to effect the desired assay sequence. For another, the size of each of the wells is set so as to permit the strip to be inserted. For another, the wells are deep enough to accommodate a depth of liquid "d" which is a depth adequate to completely cover all the readable areas 12, 14 and 15; or 12, 14, 24 and 15, present on the test strip.

In use, one or more of the wells of test plate 22 are charged with predetermined quantities of reagents and samples according to a predetermined protocol and then the test strip is inserted into the wells, again according to a predetermined protocol. This gives rise to a readable detection event which is read together with the results of the positive and negative controls. On the basis of these readings, one may determine whether a valid test has been conducted and whether the analyte is present in the specimen.

For example, in a typical protocol a test strip might be constructed with an antibody against a suspected pathogen in human blood. Positive control 15 would have an antibody against a human antibody common in blood, and negative control 12 would have an antibody against a material not commonly found in blood but of concern as a contaminant, or the like.

Well 25 is charged with a premeasured quantity of diluent, buffer or a like aqueous medium. A given quantity of patient blood or other source is added to the liquid of well 25 and mixed with it. The strip is inserted into the liquid in well 25 for a preset interval. This permits the test and control areas on strip 21 to contact the liquid and the diluted blood constituents. This allows the immunological reactions to occur between the antibodies in these areas and any appropriate species in the blood.

The sample well 25 and any other well may contain various reagents to prevent undesired reactions and the like. These may include materials so as to alter the properties of the sample, or materials to react with or remove undesired cross-reactive materials in the samples.

A current problem with most immunodiagnostic assays is the undesirable interaction of sample constituents with the coated solid phase (i.e. background). These types of interaction often cause negative samples to be interpreted as false positives. Background may be minimized by partitioning the undesirable reactants (specific and nonspecific) onto a second solid phase (usually be preincubating the sample before exposure to the test solid phase). In the case of antibody capture assays, the second solid phase would have bound to it antibody specific for an analyte not found in the sample or test (i.e., irrelevant specificity). This type of second solid phase would remove both specific and nonspecific background constituents for antibodies, the solid phase, the blocking reagent, or a combination of any of the three. The second solid phase may be used in any or all parts of the assay although preferably eliminated at the "developer" stage. In the case of human derived viral components as analyte, preinfected human components may be used as adsorbent reagent for the second solid phase partitioning.

The configuration for the second phase should be designed for the mechanics of the reaction. Adsorbent reagents would coat the chamber walls of the sample tray for some solutions. For rapid immunoassay, a critical step in controlling background is the initial mixing of solid phase and sample. The second solid phase should be free to move within the mixing solution (a second strip of any geometric shape or particles). A preferred method would employ an adsorbing means comprising a suspension of particle beads (approximate diameter of 1 to 10 micrometers) or any or all of the chambers into which the test strip is immersed. The advantage is a large total surface area for rapid reaction kinetics and maximum diffusion of reactants. Preincubation of sample with adsorbent coated particles would quickly remove background components before exposure to the test strip. Preparation of these types of particles is described in the examples.

Next the test strip is removed from well 25 and inserted into well 26. Well 26 may contain, for example, a premeasured quantity of a wash solution to remove interfering materials, or the like. Thereafter, the test strip is passed to a well 27 containing a detection system, for example, a labeled antigen, or a labeled antibody to the bound antigen or the like. Next, the strip may be moved to well 29 for washing and finally to well 30 where a substrate (e.g., chromogenic reagent) is added or already present. In some cases, excess liquid on the strip may be removed and substrate added directly to the strip. Other wells may be present to contain other reagents as required or desired.

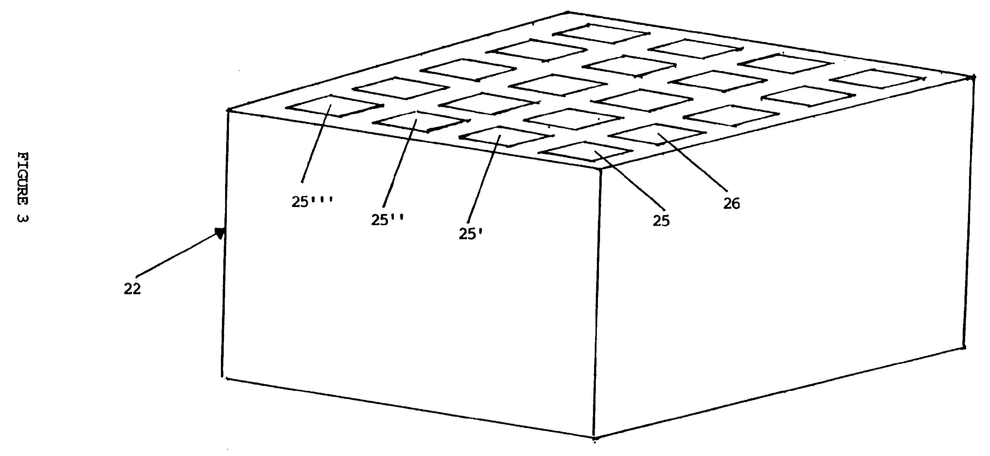

Figure 3 illustrates an extension of the invention in which test block 22 includes a plurality of sets (i.e., 4 sets) of wells so that there are four sample wells 25, four wash wells 26, etc. This permits multiple samples to be tested at once with multiple test strips.

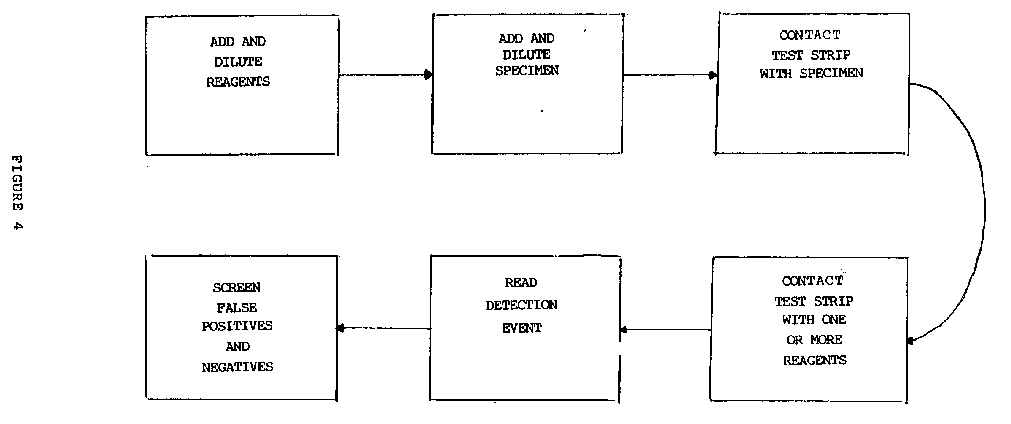

Figure 4 provides a block diagram of the typical test protocol just set forth and points out the sequential nature of the test method.

It will be appreciated by those skilled in the art that this invention is not limited to particular antibodies, reagents, particular samples, or particular utilization chemistries and that reasonable alternatives may be employed without departing from its teachings.

It should be noted that suitable antibodies for use in the test reagent region(s) may be monoclonal antibodies, polyclonal antibodies, mono- or divalent antibody fragments, hybrid antibodies, heterobi-functional antibodies, or genetically manipulated or cloned antibodies.

The test itself is, as noted above, a method of immunologically detecting the presence of one or more analytes in a specimen. Typically, the test is used in either the detection or the quantitation of bound or unbound label, wherein the amount of label detected corresponds to the amount of analyte in the specimen. As used herein, the "immunoassay procedure" of the subject invention may be in the nature of immuno-electron microscopy of fluroescence polarization, or it may be an immuno-fluorescent or fluoroescent immunoassay, a radiometric assay, an enzyme-linked immunoassay, or a photon-counting bioluminescent or chemiluminescent assay. In the case of the enzyme-linked immunoassay, the enzyme used is normally selected from the group consisting of alkaline phosphatase, glucose oxidase, horseradish peroxidase, urease, luciferase, and galactosidase. The label itself may be virtually any detectable species, e.g., a chromogenic compound, a radioactive isotope, an enzyme, or a fluorescent, luminescent, bioluminescent, chemiluminescent, phosphorescent, or ferromagnetic material.

The analyte may be virtually any compound or organism which is detectable using the aforementioned procedure, i.e., a drug, hormone, vitamin, enzyme, ligand, protein, including glycoprotein and lipoprotein, antibody, polysaccharide, cell or tissue antigen, or bacterium, protozoon, parasite, fungus, virus, blood cell substance, blood fluid substance, or a component of any of the foregoing. In a preferred embodiment, a plurality of analytes is detected, which in a particularly preferred embodiment includes HIV-1 GAG protein, HIV-1 ENV glycoprotein, HIV-1 POL protein, HTLV-1 GAG protein, HTLV-1 ENV glycoprotein, and HTLV-1 POL protein purified from natural isolates, recombinant genetic manipulations or chemical synthesis.

The aforementioned analytes may also be bound to the test strip as the actual detecting reagents in alternative assay procedures.

The specimen may be blood or its fractionated components, urine, saliva, vaginal fluid, seminal fluid, mucosa, birth fluids, tears, gastrointestinal fluid and excrement, inflammatory fluids, pleural effusion, pulmonary fluid, tissue extracts and the like derived from mammalian, avian, reptilian, amphibian, arthropod and the like.

The invention is further illustrated by the following examples which are not intended to and should not be construed so as to limit in any way the scope of the invention as defined by the claims which follow thereafter.

### EXAMPLE 1

### Detection of Human Anti-HIV-1 Antibody and HIV-1 Antigen in One Assay Minimization of False Positives

### I. Preparation of the Test Stick

Test strip prototypes were constructed from the lids of 96-well tissue culture plates (Costar, catalog #3096). Strips were cut from the lid using a sharp knife attachment on a soldering iron so that the strip contained 8 contiguous circles, each surrounded by a raised peripheral ring. Strips can also be used as support segments to contain highly adsorbent solid phase (e.g., Nylon membrane or nitrocellulose paper). The strip was washed in a buffer and allowed to air dry.

Monoclonal antibodies, designated DNP1 (CB6 mouse IgG1, specific for dinitrophenol) and anti-HIgG (CB6 mouse IgG1, specific for human immunoglobulin G) were gifts from Dr. Pao-Min Loh's laboratory (University of Iowa). Anti-HIV GAG (mouse IgG1, specific for Human Immunodeficiency Virus-1) was purchased from Epitope, Inc. (catalog #5001). Cocktails of MAbs with specificity for each analyte can be used. Here, the MAbs were made 100 micrograms/ml in Tris Buffered Saline (TBS, 0.15 M NaCl, 0.05 M Tris, pH 7.2) and 10 microliters was applied directly to designated circular segments. HIV-1 (Litton Bionetics, HTLV-3, cat. #37225, 1 milligram/ml, NP40 inactivated) was made 2 micrograms/ml in TBS. 10 microliters were applied directly to the designated circular segment. The ordering of reagents was as follows:
Anti-human IgG (positive control) [top]
Anti-HIV GAG (detects virus antibody complex)
HIV-1 (detects antibody)
Anti-DNP (negative control) [bottom]

The stick was placed horizontally (solution side up) into a 45°C oven and the antibody and antigen allowed to dry for about 30 minutes. The stick was placed in blocking solution (TBS with 0.3% Tween®, 1 mg/ml bovine serum albumin, BSA, and 0.1% sodium azide) for a minimum of 15 minutes.

### II. Preparation of Adsorbent Beads

To minimize the contribution of certain samples' affinity to bind to proteins denatured on solid surfaces (or solid surfaces directly), latex polystyrene particles were covalently coupled with monoclonal anti-DNP antibody (purified human T-cell plasma membrane can also be coupled to latex particles when viral analyte is either directly adsorbed or chemically coupled). In addition, the nonspecific adsorbent can be coated on any other solid phase or directly onto the walls of the chamber. Antibody is made 200 micrograms/ml in PBS (0.1 M phosphate-buffered saline, pH 7.2, Pandex Laboratories published Research Report, No. 4 (1984) "Coupling of Antigens to Latex Particles by the Water-Soluble Carbodiimide Method," by Michael E. Jolley, Ph.D.). The antibody solution was added to washed and pelleted latex particles (Pandex, Epicon carboxyl-polystyrene particles, catalog #31-010-1). The solution was triturated and exposed to 20 sec of an ultrasonic water bath. The final concentration of beads was 0.5%. Solid carbodiimide (Sigma, 1-ethyl-3-[3-dimethylaminopropyl]-carbodiimide, catalog #E-7750) is added to make a final concentration of about 5 milligrams/ml. The solution is rotated gently for one hour at room temperature and washed (with PBS) and pelleted (12,000 x g, 3 min. room temperature) three times. The final bead solution is made 0.01% to 0.5% (w/v) in blocking buffer.

### III. Sample Tray

A sample tray was configured by solvent welding plastic cuvettes (Elkay Products, catalog #127-1010-400) with a brand cyanocrylate glue. The cuvettes were 1 cm x 1 cm x 4.5 cm. In this example, the tray contains 7 cuvettes (with lids). In this example, the first compartment contained 2.5 ml of an approximately 1.0 micrograms/ml, inactivated HIV-1 (Litton Bionetics, HTLV-3, cat. #37225, 1 milligram/ml, NP40 inactivated) in blocking buffer container about 0.1% adsorbent beads described above. The second compartment contained 2.5 milliliters wash solution (TBS/0.3% Tween). The third compartment contained a mixing solution (2.5 milliliters of blocking solution with about 0.1% adsorbent beads) for diluting the human blood specimen. The fourth compartment contained 2.5 milliliters wash solution (TBS/0.3% Tween®). The fifth compartment contained 2.5 milliliters of developing conjugate solution; polyclonal goat anti-human immunoglobulin coupled to alkaline phosphatase (Jackson Immunoresearch Labs, catalog #109-5576) diluted 1:500 in blocking solution with about 0.1% adsorbent beads. The sixth compartment contained 2.5 milliliters of wash solution (TBS/0.3% Tween®). The seventh compartment contained 2.5 milliliters of presubstrate wash solution (pH 10 alkaline buffer).

### IV. The Assay

The lid for chamber #3 was removed. A drop of blood (obtained by a lancet puncture to a finger alternatively, 1 to 50 microliters of serum may be used) was allowed to drip into the third compartment. With each chamber covered with its respective lid, the entire tray was gently shaken several times to ensure mixing of blood and bead solution. The tray was returned to its original standing position and the lid removed from chamber #1. While the blood was incubating in chamber #3, a test strip was immersed in chamber #1. The solution was gently stirred with the test strip and allowed to incubate at 42°C for about 20 minutes. Care was taken to ensure that the readable was maximally exposed to the solvent phase. The strip was transferred to compartment #2 and washed for about 10 seconds. The strip was transferred to compartment #3 containing the blood/adsorbent mixture. The solution was gently mixed and allowed to incubate with the strip for about 30 minutes at 42°C. The strip was transferred to compartment #4 and washed for about 10 seconds. The strip was transferred to compartment #5. With about 5 seconds of gentle stirring, the strip was allowed to incubate (stirring every 1 min.). After a total of about 10 minutes incubation, the strip was transferred to compartment #6 and gently stirred for about 10 seconds. The strip was transferred to compartment #7 and washed for about 10 seconds. The strip was removed and tapped lightly to remove excess liquid. One drop (about 50 microliters) of substrate (5-bromo-4-chloro-3-indolyl phosphate, Sigma catalog #B-0766, in alkaline buffer, pH 10) was added to each readable area. After approximately 10 minutes, the color reaction of each readable area was recorded as positive (blue color) or negative (no color).

### V. Interpretation

The assay can only be valid if the segment containing the anti-DNP MAb (negative control) remains colorless. The adsorbent beads should remove all materials that cause a false positive reaction and the negative control readable area was used to confirm this function. The anti-HIg segment should bind human immunoglobulin present in blood samples. This reaction must be positive or else the test reagents are not working, thus invalidating the test. A positive blue color in the HIV-1 segment indicates that there is antibody to HIV-1 present in the blood sample. A positive response in the anti-HIV segment suggests that virus is present complexed with human anti-HIV antibody.

### EXAMPLE 2

### Detection of Human Anti-HIV-1 Antibody Minimization of False Positives

### I. Preparation of the Test Stick

Test strip prototypes were constructed from the lids of 96-well tissue culture plates (Costar, catalog #3096). Strips were cut from the lid using a sharp knife attachment on a soldering iron so that the strip contained 8 contiguous circles, each surrounded by a raised peripheral ring. Strips can also be used as support segments to contain highly adsorbent solid phase (e.g., Nylon membrane or nitrocellulose paper), also, sticks can be constructed in such a way that can contain recessed reactive areas. The strip was washed in a buffer and allowed to air dry.

Monoclonal antibodies, designed DNP1 (CB6 mouse IgG1, specific for dinitrophenol) and anti-HIgG (CB6 mouse IgG1, specific for human immunoglobulin G) were used. Cocktails of MAbs with specificity for each analyte can be used. Here, the MAbs were made 100 micrograms/ml in Tris Buffered Saline (TBS, 0.15 M NaCl, 0.05 M Tris, pH 7.2) and 10 microliters applied directly to designated circular segments. HIV-1 (Litton Bionetics, HTLV-3, cat. #37225, 1 milligram/ml, NP40 inactivated) was made 2 micrograms/ml in TBS. 10 microliters were applied directly to the designated circular segment. The ordering of reagents was as follows:
Anti-human IgG (positive control) [top]
HIV-1 (detects antibody) [middle]
Anti-DNP (negative control) [bottom]

The stick was placed horizontally (solution side up) into a 45°C oven and the antibody allowed to dry for about 30 minutes. The stick was placed in blocking solution (TBS with 0.3% Tween®, 1 mg/ml bovine serum albumin, BSA, and 0.1% sodium azide) for a minimum of 15 minutes.

### II. Preparation of Adsorbent Beads

To minimize the contribution of certain samples' affinity to bind to proteins denatured on solid surfaces (or solid surfaces directly), latex polystyrene particles were covalently coupled with monoclonal anti-DNP antibody (purified human T-cell plasma membrane or recombinant DNA related proteins such as beta-galactosidase can also be coupled to latex particles when viral analyte is either directly adsorbed or chemically coupled). In addition, the non-specific adsorbent can be coated on any other solid phase or directly onto the walls of the chamber. Antibody is made 200 micrograms/ml in PBS (0.1 M phosphate-buffered saline, pH 7.2, Pandex Laboratories published Research Report, No. 4 (1984) "Coupling of Antigens to Latex Particles by the Water-Soluble Carbodiimide Method," by Michael E. Jolley, Ph.D.). The antibody solution was added to washed and pelleted latex particles (Pandex, Epicon carboxyl-polystyrene particles, catalog #31-010-1). The solution was triturated and exposed to 20 sec of an ultrasonic water bath. The final concentration of beads was 0.5%. Solid carbodiimide (Sigma, 1-ethyl-3-[3-dimethylaminopropyl]-carbodiimide, catalog #E-7750) is added to make a final concentration of about 5 milligrams/ml. The solution is rotated gently for one hour at room temperature and washed (with PBS) and pelleted (12,000 x g, 3 min., room temperature) three times. The final bead solution is made 0.01% to 0.5% (w/v) in blocking buffer.

### III. Sample Tray

A sample tray was configured by solvent welding plastic cuvettes (Elkay Products, catalog #127-1010-400) with a cyanoacrylate glue. The cuvettes were 1 cm x 1 cm x 4.5 cm. In this example, the tray contains 6 cuvettes (with lids). The first chamber contained a mixing solution (2.5 milliliters of blocking solution with about 0.1% adsorbent beads) for diluting the human blood specimen. The second chamber contained 2.5 milliliters wash solution (TBS/0.3% Tween®). The third chamber contained 2.5 milliliters of developing conjugate; polyclonal goat anti-human immunoglobulin coupled to alkaline phosphatase (Jackson Immunoresearch Labs, catalog #109-5576) diluted 1:500 in blocking solution. The fourth chamber contained 2.5 milliliters of wash solution (TBS/0.3% Tween). The fifth chamber contained 2.5 milliliters of presubstrate wash solution (pH 10 alkaline buffer). The sixth chamber contained 1 ml of substrate solution (5-bromo-4-chloro-3-indolyl phosphate, Sigma catalog #B-0766, in alkaline buffer, pH10).

### IV. The Assay

The sample tray was equilibrated in a 42° water bath. 5 microliters (5-200 microliters can be used) of serum or plasma was added into the first chamber. The sample in chamber 1 was allowed to incubate for 3 minutes at 42°C. (40-45°C may be used). The solution was gently stirred with the test strip and allowed to incubate at 42°C for about 45 minutes. Care was taken to ensure that the readable area was maximally exposed to the solvent phase. The strip was transferred to chamber #2 and washed for about 1-10 seconds. The strip was transferred to chamber #3. With about 5 seconds of gentle stirring, the strip was allowed to incubate at 42°C for about 45 minutes. The strip was transferred to chamber #4 and gently stirred for about 1-10 seconds. The strip was transferred to chamber #5 and washed for about 1-10 seconds. The entire sample tray was removed from the 42°C water bath and placed on the bench at room temperature. The strip was transferred to chamber #6 and allowed to incubate at room temperature for 10-20 minutes. The color reaction of each readable area was recorded as positive (blue color) or negative (no color).

Three double-blind studies were performed to determine the presence of antibodies to HIV-1 in patient serum.

Test A: 25 out of 50 serum samples (Peralta Cancer Center, Oakland, CA) were Western Blot (WB) positive for HIV-1 antibodies (predominantly gp41 only). The other 25 were from a WB negative donor serum pool. The test accurately identified 100% of the samples as negative or positive as compared with the WB results.

Test B: 21 HIV-1 WB positive blood samples, and 7 false positive blood samples (as originally screened on the Abbott HIV-1 antibody test at the Massachusetts General Hospital Blood Bank, Boston, MA) were tested with the test. In the test, all 21 WB positive samples were confirmed along with the 23 WB negative samples. All 7 false positive samples were negative by the test.

Test C: 9 HIV-1 WB positive blood samples (NYU Medical Center) and 10 WB negative blood samples were correctly identified by the test. Two HIV-1 antibody positive semen samples, two saliva samples and 1 urine sample were also positive in the test.

### EXAMPLE 3

### Detection of HIV-1 P24 Antigen in Patient's Serum

### I. Preparation of the Test Sticks

Test sticks are the same as those in Example 1. The following monoclonal antibodies are adsorbed to the stick: 1F8 (Calypte Biomedical Company's anti HIV-1 GAG monoclonal antibody, IgG1); anti-HIgG and anti-DNP. Here, the MAbs were made 100 milligram/ml in Tris Buffered Saline and 10 microliters applied directly to a designated circular segment The ordering of MAbs was as follows:
Anti-Human IgG (positive control) [top]
Anti-HIV GAG (detects P24) [middle]
Anti-DNP (negative control) [bottom]

The stick is placed horizontally solution side up into a 45°C oven and antibodies allowed to adsorb for about 60 minutes. The stick was placed in blocking solution (TBS, 5% horse serum) for a minimum of 15 minutes.

### II. Preparation of the Adsorbent Beads

To minimize the contribution of certain samples' affinity to bind to proteins denatured on solid surfaces (or solid surfaces directly) and to affect removal of contaminating human anti-GAG (soluble or in complexes), latex polystyrene particles were covalently coupled with recombinant HIV-1 p24 GAG protein. The recombinant antigen is made 100-200 micrograms/ml in PBS. The antigen solution was added to washed and pelleted latex particles. The solution was triturated and exposed for 20 seconds of an ultrasonic bath. The final concentration of beads was 0.5%. Solid carbodiimide is added to make a final concentration of about 5 milligrams per ml. The solution is rotated gently for one hour at room temperature and washed (with PBS) and pelleted (12,000 x g) three minutes, room temperature 3 times. The final bead solution is made 0.01% to 0.5% (w/v) in blocking buffer.

### III. Sample Tray

The first compartment contained 2.5 ml of blocking buffer containing about 0.1% recombinant antigen adsorbent beads described above. The second compartment contained 2.5 milliliters wash solution (TBS). The third compartment contained 2.5 milliliters of developing conjugate solution; polyclonal goat anti-HIV-1 GAG coupled to alkaline phosphatase diluted 1:500 in blocking solution. The fourth compartment contained 2.5 milliliters of wash solution (TBS). The fifth compartment contained 2.5 milliliters of presubstrate wash solution (ph 10 alkaline buffer).

### IV. The Assay

100 microliters of patient sample (blood, serum, plasma) is added to a shallow well, containing twenty microliters of dissociation buffer (1 M HCl-glycine buffer). The solution is then transferred into chamber #1. The solution was gently stirred with the test strip and allowed to incubate at 42°C for about 20 minutes. Care was taken to ensure that the readable area was maximally exposed to the solvent phase. The strip was transferred to compartment #2 and washed for about 10 seconds. The strip was transferred to compartment #3 with about five seconds of gentle stirring. The strip was allowed to incubate for about 20 minutes at 42°C. The strip was transferred to compartment #4 and gently stirred for about 10 seconds. The strip was transferred to compartment #5 and washed for about 10 seconds. The strip was removed and tapped lightly to remove excess liquid. One drop (about 50 microliters) of substrate (5-bromo-4-chloro-3-indolyl phosphate, Sigma catalog #B-0766, in alkaline buffer, pH 10) was added to each readable area. After approximately 10 minutes, the color reaction of each readable area was recorded as positive (blue color) or negative (no color).

## Claims

1. A test strip for detecting, in a sample from a human subject, the presence of an antigenic substance which comprises:
a) a solid support;
b) an antibody-based reagent directed against the antigenic substance which substance is in an immune complex with a native human antibody thereto, bound to a first discrete area on the solid support;
c) an anti-(human antibody) antibody bound to a second discrete area on the solid support as a positive control; and
d) an antibody directed against an antigen which does not naturally occur in human subjects bound to a third discrete area on the solid support as a negative control.

2. A test strip of claim 1, wherein the antigenic substance is a virus or a viral protein.

3. A test strip of claim 2, wherein the antigenic substance is HIV-1, HIV-1 GAG protein, HIV-1 ENV glycoprotein, HIV-1 POL protein, HTLV-1 GAG protein, HTLV-1 ENV glycoprotein, HTLV-1 POL protein, or an epitope thereof.

4. A test strip of any of claims 1 to 3, wherein the solid support comprises glass fiber filter paper, nitrocellulose, scintered glass, plastic, synthetic polymer, cellulose, cellulose acetate, polytetrafluoroethylene, polyethylene, polypropylene, or polyvinylidine fluoride.

5. A test strip of any of claims 1 to 4, wherein the antibody-based reagent comprises a monoclonal antibody, polyclonal antibody, mono- or divalent antibody fragment, hybrid antibody, heterofunctional antibody, or genetically manipulated or cloned antibody.

6. A test strip of any of claims 1 to 5, wherein the anti-(human antibody) antibody is a monoclonal antibody.

7. A test strip of any of claims 1 to 6, wherein the antigen which does not naturally occur in human subjects is a synthetic organic molecule.

8. A test strip of claim 7, wherein the synthetic organic molecule is dinitrophenol.

9. A method for detecting in a sample from a human subject the presence of an antigenic substance, which substance is in an immune complex with a native human antibody, which comprises:
a) pretreating the sample to be tested so as to prevent non-specific and specific binding of substances present in the sample to proteins, including antibodies, present on the test strip and thus prevent spurious results;
b) contacting the resulting pretreated sample with the test strip of any of claims 1 to 8 under conditions such that the antigenic substance present in the sample binds to the antibody-based reagent thereto present on the test strip;
c) thereafter treating the test strip to remove sample;
d) contacting the resulting, treated test strip with labeled antibody under conditions such that the labeled antibody forms a complex with any antigenic substance bound to the test strip;
e) detecting the presence of labeled antibody bound to the test strip, and thereby the presence of the antigenic substance in the sample; and
f) verifying the correctness of the detection so made by means of the positive and negative controls on the test strip by contacting the test strip with labeled anti human-antibody antibody under conditions such that the labeled antihuman-antibody antibody binds to human antibody bound to the test strip.

10. A method of claim 9 or 10, wherein the antigenic substance is a virus or viral protein.

11. A method of claim 9 or 10, wherein the sample is blood, serum, or urine.

12. A test strip for detecting, in a sample from a human subject, the presence of an antibody which comprises:
a) a solid support;
b) an antigenic substance which binds to the antibody bound to a first discrete area on the solid support;
c) an anti-(human antibody) antibody bound to a second discrete area on the solid support as a positive control; and
d) an antibody directed against an antigen which does not naturally occur in human subjects bound to a third discrete area on the solid support as a negative control.

13. A test strip for detecting, in a sample from a human subject, the presence of an antigenic substance and an antibody which comprises:
a) a solid support;
b) an antigenic substance which binds to the antibody bound to a first discrete area on the solid support;
c) an antibody directed against the antigenic substance of the sample, which substance is in an immune complex with a native human antibody, bound to a second discrete area on the solid support;
d) an anti-(human antibody) antibody bound to a third discrete area on the solid support as a positive control;
e) an antibody directed against an antigen which does not naturally occur in human subjects bound to a fourth discrete area on the solid support as a negative control.

14. A method for detecting, in a sample from a human subject, the presence of an antibody which comprises:
a) pretreating the sample to be tested so as to prevent non-specific and specific binding of substances present in the sample to proteins, including antibodies and antigens, present on the test strip and thus prevent spurious results;
b) contacting the resulting pretreated sample with the test strip of claim 12 under conditions such that the antibody present in the sample binds to the antigenic substance present on the test strip;
c) thereafter treating the test strip to remove sample;
d) contacting the resulting, treated test strip with labeled antihuman-antibody antibody under conditions such that the antihuman-antibody antibody forms a complex with any human antibody bound to the test strip;
e) detecting the presence of labeled antihuman-antibody antibody bound to the test strip in the first discrete area and thereby the presence of the antibody in the sample; and
f) verifying the correctness of the detection so made by means of the positive and negative controls on the test strip.

15. A method for detecting, in a sample from a human subject, the presence of an antigenic substance and an antibody which comprises:
a) pretreating the sample to be tested so as to prevent non-specific and specific binding of substances present in the sample to proteins, including antibodies and antigens, present on the test strip and thus prevent spurious results;
b) contacting the resulting pretreated sample with the test strip of claim 13 under conditions such that the antibody binds to the antigenic substance present on the test strip and the antigenic substance of the sample binds to the antibody present on the test strip;
c) thereafter treating the test strip to remove sample;
d) contacting the resulting, treated test strip with labeled antibody to the antigenic substance of the sample and with labeled antihuman-antibody antibody under conditions such that labeled antibody forms a complex with the antigenic substance bound to the test strip and the labeled antihuman-antibody antibody forms a complex with any human antibody bound to the test strip;
e) detecting the presence of labeled antibody and labeled antihuman-antibody antibody bound to the test strip, and thereby the presence of the antigenic substance and the antibody in the sample; and
f) verifying the correctness of the detection so made by means of the positive and negative controls on the test strip.

## Patentansprüche

1. Teststreifen zum Nachweis der Anwesenheit einer antigenen Substanz in einer einem Menschen entnommenen Probe, umfassend:
a) einen festen Schichtträger;
b) ein gegen die antigene Substanz, die in einem Immunkomplex mit einem nativen Humanantikörper gegen sie vorliegt, gerichtetes Reagenz auf Antikörperbasis, welches an einen ersten abgesonderten Bereich auf dem festen Schichtträger gebunden ist;
c) einen Anti-(Humanantikörper)Antikörper, welcher als positive Kontrolle an einen zweiten abgesonderten Bereich auf dem festen Sichtträger gebunden ist und
d) einen gegen ein bei Menschen nicht natürlich vorkommendes Antigen gerichteten Antikörper, welcher als negative Kontrolle an einen dritten abgesonderten Bereich auf dem festen Schichtträger gebunden ist.

2. Teststreifen nach Anspruch 1, wobei die antigene Substanz aus einem Virus oder Virusprotein besteht.

3. Teststreifen nach Anspruch 2, wobei die antigene Substanz aus HIV-1, HIV-1 GAG-Protein, HIV-1 ENV-Glycoprotein, HIV-1 POL-Protein, HTLV-1 GAG-Protein, HTLV-1 ENV-Glycoprotein, HTLV-1 POL-Protein oder einem Epitop hiervon besteht.

4. Teststreifen nach einem der Ansprüche 1 bis 3, wobei der feste Träger Glasfaserfilterpapier, Nitrocellulose, gefrittetes Glas, Kunststoff, ein synthetisches Polymer, Cellulose, Celluloseaceat, Polytetrafluorethylen, Polyethylen, Polypropylen oder Polyvinylidenfluorid umfaßt.

5. Teststreifen nach einem der Ansprüche 1 bis 4, wobei das Reagens auf Antikörperbasis einen monoklonalen Antikörper, einen polyklonalen Antikörper, ein ein- oder zweiwertiges Antikörperfragment, einen Hybridantikörper, einen heterofunktionellen Antikörper oder einen genetisch manipulierten oder geklonten Antikörper umfaßt.

6. Teststreifen nach einem der Ansprüche 1 bis 5, wobei der Anti-(Humanantikörper)Antikörper aus einem monoklonalen Antikörper besteht.

7. Teststreifen nach einem der Ansprüche 1 bis 6, wobei das bei Menschen nicht natürlich vorkommende Antigen aus einem synthetischen organischen Molekül besteht.

8. Teststreifen nach Anspruch 7, wobei das synthetische organische Molekül aus Dinitrophenol besteht.

9. Verfahren zum Nachweis der Anwesenheit einer in einem Immunkomplex mit einem nativen Humanantikörper vorliegenden antigenen Substanz in einer einem Menschen entnommenen Probe durch:
a) Vorbehandeln der zu testenden Probe zur Verhinderung einer unspezifischen und spezifischen Bindung von in der Probe enthaltenen Substanzen an auf dem Teststreifen vorhandenen Proteinen einschließlich Antikörpern und somit zur Verhinderung falscher Ergebnisse;
b) Kontaktieren der erhaltenen vorbehandelten Probe mit dem Teststreifen nach einem der Ansprüche 1 bis 8 unter solchen Bedingungen, daß die in der Probe enthaltene antigene Substanz an das auf dem Teststreifen vorhandene Reagenz auf der Basis eines gegen sie gerichteten Antikörpers gebunden wird;
c) anschließendes Behandeln des Teststreifens zur Entfernung der Probe;
d) Kontaktieren des erhaltenen behandelten Teststreifens mit einem markierten Antikörper unter solchen Bedingungen, daß der markierte Antikörper mit irgendeiner an den Teststreifen gebundenen antigenen Substanz einen Komplex bildet;
e) Nachweisen der Anwesenheit des an den Teststreifen gebundenen markierten Antikörpers und dadurch der Anwesenheit der antigenen Substanz in der Probe und
f) Verifizieren der Richtigkeit des derart durchgeführten Nachweises mit Hilfe der positiven und negativen Kontrollen auf dem Teststreifen durch Kontaktieren des Teststreifens mit einem markierten Anti-(Humanantikörper)Antikörper unter solchen Bedingungen, daß der markierte Anti-(Humanantikörper)Antikörper an den an den Teststreifen gebundenen Humanantikörper gebunden wird.

10. Verfahren nach Anspruch 9 oder 10, wobei die antigene Substanz aus einem Virus oder Virusprotein besteht.

11. Verfahren nach Anspruch 9 oder 10, wobei die Probe aus Blut, Serum oder Urin besteht.

12. Teststreifen zum Nachweis der Anwesenheit eines Antikörpers in einer einem Menschen entnommenen Probe, umfassend:
a) einen festen Schichtträger;
b) eine antigene Substanz, die eine Bindung mit dem an einen ersten abgesonderten Bereich auf dem festen Schichtträger gebundenen Antikörper eingeht;
c) einen Anti-(Humanantikörper)Antikörper, welcher als positive Kontrolle an einen zweiten abgesonderten Bereich auf dem festen Schichtträger gebunden ist und
d) einen gegen ein bei Menschen nicht natürlich vorkommendes Antigen gerichteten Antikörper, welcher als negative Kontrolle an einen dritten abgesonderten Bereich auf dem festen Schichtträger gebunden ist.

13. Teststreifen zum Nachweis der Anwesenheit einer antigenen Substanz und eines Antikörpers in einer einem Menschen entnommenen Probe, umfassend:
a) einen festen Schichtträger;
b) eine antigene Substanz, die eine Bindung mit dem an einen ersten abgesonderten Bereich auf dem festen Schichtträger gebundenen Antikörper eingeht;
c) einen gegen die in einem Immunkomplex mit einem nativen Humanantikörper vorliegende antigene Substanz der Probe gerichteten Antikörper, welcher an einen zweiten abgesonderten Bereich auf dem festen Schichtträger gebunden ist;
d) einen Anti-(Humanantikörper)Antikörper, welcher als positive Kontrolle an einen dritten abgesonderten Bereich auf dem festen Sichtträger gebunden ist;
e) einen gegen ein bei Menschen nicht natürlich vorkommendes Antigen gerichteten Antikörper, welcher als negative Kontrolle an einen vierten abgesonderten Bereich auf dem festen Schichtträger gebunden ist.

14. Verfahren zum Nachweis der Anwesenheit eines Antikörpers in einer einem Menschen entnommenen Probe durch:
a) Vorbehandeln zu testenden Probe zur Verhinderung einer unspezifischen und spezifischen Bindung von in der Probe enthaltenen Substanzen an auf dem Teststreifen vorhandene Proteine einschließlich Antikörper und Antigene und somit zur Verhinderung falscher Ergebnisse;
b) Kontaktieren der erhaltenen vorbehandelten Probe mit dem Teststreifen nach Anspruch 12 unter solchen Bedingungen, daß der in der Probe enthaltene Antikörper an die auf dem Teststreifen vorhandene antigene Substanz gebunden wird;
c) anschließendes Behandeln des Teststreifens zur Entfernung der Probe;
d) Kontaktieren des erhaltenen behandelten Teststreifens mit einem markierten Anti-(Humanantikörper)-Antikörper unter solchen Bedingungen, daß der Anti-(Humanantikörper)Antikörper mit irgendeinem an den Teststreifen gebundenen Humanantikörper einen Komplex bildet;
e) Nachweisen der Anwesenheit eines an den Teststreifen in dem ersten abgesonderten Bereich gebundenen markierten Anti-(Humanantikörper)Antikörper und dabei der Anwesenheit des Antikörpers in der Probe und
f) Verifizieren der Richtigkeit des derart durchgeführten Nachweises mit Hilfe der positiven und negativen Kontrollen auf dem Teststreifen.

15. Verfahren zum Nachweis der Anwesenheit einer antigenen Substanz und eines Antikörpers in einer einem Menschen entnommenen Probe durch:
a) Vorbehandeln der zu testenden Probe zur Verhinderung einer unspezifischen und spezifischen Bindung von in der Probe enthaltenen Substanzen an auf dem Teststreifen vorhandene Proteine einschließlich Antikörper und Antigene und somit zur Verhinderung falscher Ergebnisse;
b) Kontaktieren der erhaltenen vorbehandelten Probe mit dem Teststreifen nach Anspruch 13 unter solchen Bedingungen, daß der Antikörper an die auf dem Teststreifen vorhandene antigene Substanz gebunden wird und die antigene Substanz der Probe an den auf dem Teststreifen vorhandenen Antikörpern gebunden wird;
c) anschließendes Behandeln des Teststreifens zur Entfernung der Probe;
d) Kontaktieren des erhaltenen behandelten Teststreifens mit einem markierten Antikörper gegen die antigene Substanz der Probe und mit einem markierten Anti-(Humanantikörper)Antikörper unter solchen Bedingungen, daß der markierte Antikörper mit der an den Teststreifen gebundenen antigenen Substanz einen Komplex bildet und der markierte Anti-(Humanantikörper)Antikörper mit irgendeinem an den Teststreifen gebundenen Humanantikörper einen Komplex bildet;
e) Nachweisen der Anwesenheit des an den Teststreifen gebundenen markierten Antikörpers und markierten Anti-(Humanantikörper)Antikörpers und dabei der Anwesenheit der antigenen Substanz und des Antikörpers in der Probe und
f) Verifizieren der Richtigkeit des derart durchgeführten Nachweises mit Hilfe der positiven und negativen Kontrollen auf dem Teststreifen.

## Revendications

1. Bandelette test destinée à la détection de la présence d'une substance antigénique dans un échantillon émanant d'un sujet humain, laquelle comprend:
a) un support solide,
b) un réactif à base d'anticorps dirigé contre la substance antigénique, laquelle substance est en complexe immun avec un anticorps humain natif dirigé contre elle, lié à une première zone discrète du support solide,
c) un anticorps anti-anticorps humain lié à une deuxième zone discrète du support solide en tant que témoin positif et
d) un anticorps dirigé contre un antigène qui n'existe pas naturellement chez les sujets humains, lié à une troisième zone discrète du support solide en tant que témoin négatif.

2. Bandelette test selon la revendication 1, dans laquelle la substance antigénique est un virus ou une protéine virale.

3. Bandelette test selon la revendication 2, dans laquelle la substance antigénique est HIV-1, la protéine GAG de HIV-1, la glycoprotéine ENV de HIV-1, la protéine POL de HIV-1, la protéine GAG de HTLV-1, la glycoprotéine ENV de HTLV-1, la protéine POL de HTLV-1 ou un épitope de celles-ci.

4. Bandelette test selon l'une quelconque des revendications 1 à 3, dans laquelle le support solide comprend fibre de verre, papier filtre, nitrocellulose, verre fritté, plastique, polymère synthétique, cellulose, acétate de cellulose, polytétrafluoroéthylène, polyéthylène, polypropylène ou poly(fluorure de vinylidène).

5. Bandelette test selon l'une quelconque des revendications 1 à 4, dans laquelle le réactif à base d'anticorps comprend un anticorps monoclonal, un anticorps polyclonal, un fragment d'anticorps monovalent ou divalent, un anticorps hybride, un anticorps hétérofonctionnel ou un anticorps manipulé génétiquement ou cloné.

6. Bandelette test selon l'une quelconque des revendications 1 à 5, dans laquelle l'anticorps anti-anticorps humain est un anticorps monoclonal.

7. Bandelette test selon l'une quelconque des revendications 1 à 6, dans laquelle l'antigène qui n'existe pas naturellement chez les sujets humains est une molécule organique de synthèse.

8. Bandelette test selon la revendication 7, dans laquelle la molécule organique de synthèse est du dinitrophénol.

9. Procédé de détection de la présence d'une substance antigénique dans un échantillon émanant d'un sujet humain, laquelle substance est en complexe immun avec un anticorps humain natif, lequel procédé comprend les étapes consistant à:
a) prétraiter l'échantillon à doser de façon à interdire une liaison non spécifique et spécifique des substances présentes dans l'échantillon aux protéines, anticorps compris, présentes sur la bandelette test et éviter ainsi les résultats faux,
b) mettre en contact l'échantillon prétraité obtenu avec la bandelette test selon l'une quelconque des revendications 1 à 8 dans des conditions permettant à la substance antigénique présente dans l'échantillon de se lier au réactif à base d'anticorps dirigé contre elle et présent sur la bandelette test,
c) traiter ensuite la bandelette test pour retirer l'échantillon,
d) mettre en contact la bandelette test traitée obtenue avec un anticorps marqué dans des conditions permettant à l'anticorps marqué de former un complexe avec une substance antigénique liée à la bandelette test,
e) détecter la présence de l'anticorps marqué lié à la bandelette test et de ce fait la présence de la substance antigénique dans l'échantillon et
f) vérifier la justesse de la détection ainsi réalisée au moyen des témoins positif et négatif se trouvant sur la bandelette test en mettant la bandelette test en contact avec un anticorps anti-anticorps humain marqué dans des conditions permettant à l'anticorps anti-anticorps humain marqué de se lier à l'anticorps humain lié à la bandelette test.

10. Procédé selon la revendication 9 ou 10, dans lequel la substance antigénique est un virus ou une protéine virale.

11. Procédé selon la revendication 9 ou 10, dans lequel l'échantillon est du sang, du sérum ou de l'urine.

12. Bandelette test destinée à détecter la présence d'un anticorps dans un échantillon émanant d'un sujet humain, laquelle comprend:
a) un support solide,
b) une substance antigénique qui se lie à l'anticorps, liée à une première zone discrète du support solide,
c) un anticorps anti-anticorps humain lié à une deuxième zone discrète du support solide en tant que témoin positif et
d) un anticorps dirigé contre un antigène qui n'existe pas naturellement chez les sujets humains, lié à une troisième zone discrète du support solide en tant que témoin négatif.

13. Bandelette test destinée à détecter la présence d'une substance antigénique et d'un anticorps dans un échantillon émanant d'un sujet humain, laquelle comprend:
a) un support solide,
b) une substance antigénique qui se lie à l'anticorps, liée à une première zone discrète du suport solide,
c) un anticorps dirigé contre la substance antigénique de l'échantillon, laquelle substance est en complexe immun avec un anticorps humain natif, lié à une deuxième zone discrète du support solide,
d) un anticorps anti-anticorps humain lié à une troisième zone discrète du support solide en tant que témoin positif,
e) un anticorps dirigé contre un antigène qui n'existe pas naturellement chez les sujets humains, lié à une quatrième zone discrète du support solide en tant que témoin négatif.

14. Procédé de détection de la présence d'un anticorps dans un échantillon émanant d'un sujet humain, lequel comprend les étapes consistant à:
a) prétraiter l'échantillon à doser de façon à interdire la liaison spécifique et non spécifique de substances présentes dans l'échantillon aux protéines, anticorps et antigènes compris, présentes sur la bandelette test et éviter ainsi les résultats faux,
b) mettre en contact l'échantillon prétraité obtenu avec la bandelette test selon la revendication 12 dans des conditions permettant à l'anticorps présent dans l'échantillon de se lier à la substance antigénique présente sur la bandelette test,
c) traiter ensuite la bandelette test pour retirer l'échantillon,
d) mettre la bandelette test traitée obtenue en contact avec un anticorps anti-anticorps humain marqué dans des conditions permettant à l'anticorps anti-anticorps humain de former un complexe avec un anticorps humain lié à la bandelette test,
e) détecter la présence de l'anticorps anti-anticorps humain marqué lié à la bandelette test dans la première zone discrète et de ce fait la présence de l'anticorps dans l'échantillon et
f) vérifier la justesse de la détection ainsi réalisée au moyen des témoins positif et négatif se trouvant sur la bandelette test.

15. Procédé de détection de la présence d'une substance antigénique et d'un anticorps dans un échantillon émanant d'un sujet humain, lequel procédé comprend les étapes consistant à:
a) prétraiter l'échantillon à doser de façon à interdire la liaison non spécifique et spécifique de substances présentes dans l'échantillon aux protéines, anticorps et antigènes compris, présentes sur la bandelette test et éviter ainsi les résultats faux,
b) mettre en contact l'échantillon prétraité obtenu avec la bandelette test selon la revendication 13 dans des conditions permettant à l'anticorps de se lier à la substance antigénique présente sur la bandelette test, et à la substance antigénique de l'échantillon de se lier à l'anticorps présent sur la bandelette test,
c) traiter ensuite la bandelette test pour retirer l'échantillon,
d) mettre la bandelette test traitée obtenue en contact avec un anticorps marqué dirigé contre la substance antigénique de l'échantillon et avec un anticorps anti-anticorps humain marqué dans des conditions permettant à l'anticorps marqué de former un complexe avec la substance antigénique liée à la bandelette test et à l'anticorps anti-anticorps humain marqué de former un complexe avec un anticorps humain lié à la bandelette test,
e) détecter la présence de l'anticorps marqué et de l'anticorps anti-anticorps humain marqué liés à la bandelette test et de ce fait la présence de la substance antigénique et de l'anticorps dans l'échantillon et
f) vérifier la justesse de la détection ainsi réalisée au moyen des témoins positif et négatif se trouvant sur la bandelette test.
